# EUROPEAN PATENT APPLICATION

(11) **EP 4 671 991 A1**
(43) Date of publication of application: **31.12.2025**
(21) Application number: 25180832.5
(22) Date of filing: 04.06.2025
(51) Int. Cl.: G06F 9/50

(54) **ADAPTIVE COMPUTATIONAL FRAMEWORK FOR MEDICAL IMAGING APPARATUS**

(30) Priority: 28.06.2024 US 202418759402
(71) Applicant: GE Precision Healthcare LLC, Waukesha, WI 53188 (US)
(72) Inventor: MALTZ, Jonathan S., Waukesha, 53188-1696 (US); BHATIA, Parminder, Bellevue, 98004 (US); DE MAN, Bruno Kristiaan Bernard, Niskayuna, 12309-1027 (US)
(74) Representative: AWA Sweden AB

(57) **Abstract**

Methods and systems are provided for increasing an efficiency of use of computing resources of a plurality of connected imaging systems, based on adaptive prioritization of tasks within and between compute instances (108, 112, 122, 132, 142, 152, 162) and centralized task assignment. When a scan is not being performed using an imaging system, computing resources of the imaging system may be operated in a cooperative mode, where the computing resources may be advantageously used to perform processing tasks for other imaging systems of the connected imaging systems. Computing resources may be allocated based on compute time (606), data transmission time (604), and availability of compute instance (612). In particular, cloud-based AI models (800) used by an imaging system may be integrated more efficiently. For example, local versions of such models may be cached, for operation in the event of a failure of external network connections.

## Description

### TECHNICAL FIELD

Embodiments of the subject matter disclosed herein relate to medical imaging, and in particular, to managing a use of local and remote resources for medical imaging tasks.

### BACKGROUND

Medical image equipment, such as computed tomography (CT), magnetic resonance (MR), positron emission tomography (PET), and other scanners, acquire analog measurements and process the measurements to produce digital output, such as images. Typically, the analog front ends of these systems include sensors, amplifiers, signal conditioners and analog-to-digital converters. Such systems are controlled by analog and/or digital controllers. Digital controllers process digitized measurements obtained from analog components (actuator positions, motor speeds, component temperatures, x-ray tube output) and produce control outputs that may be converted to analog signals in order to, for example, move actuators, change magnetic field strength, and produce ultrasound pulse sequences.

An increasing amount of signal processing, both in the data acquisition and instrument control subsystems, is performed in the digital domain using computer programs. Examples include software-defined radio and software-defined ultrasound. As computational power has increased, and with the advent of massively parallel architectures such as GPUs, general purpose computing infrastructure (CPU- and GPU-based) has become suitable for implementing processing, vs the specialty FPGAs, DSPs and microcontrollers employed in the past. This affords the opportunity to (1) reduce the demand for custom hardware, (2) reduce the effect of device obsolescence, (3) reduce a total amount invested in computational hardware (4) reduce environmental impact of computation (power to operate, fabricate, maintain) (5) facilitate reuse of computational infrastructure for various purposes.

### SUMMARY

The current disclosure at least partially addresses one or more of the above identified issues by a resource allocation system of a connected network of medical imaging systems, the resource allocation system comprising a processor, and instructions stored in a memory of the resource allocation system that when executed, cause the processor to receive a scan protocol, reconstruction instructions, and post-reconstruction image processing instructions for a scan to be performed on a first imaging system of the connected network of medical imaging systems; perform a first set of processing tasks of the scan at one or more local compute instances (LCIs) of the first imaging system, based on the scan protocol and the reconstruction instructions; perform a second processing task of the scan at a second compute instance (CI) of the connected network of medical imaging systems, based on the post-reconstruction image processing instructions, the second processing task performed using an artificial intelligence (AI) model; and in response to a data transmission time between the first imaging system and the second CI being greater than a threshold data transmission time, sending parameter data of the AI model to a selected LCI of the one or more LCIs; reassigning the second processing task from the second CI to the selected LCI; finishing the processing of the second processing task at the selected LCI, using a fallback version of the AI model installed at the selected LCI; and displaying the processed image on a display device.

The above advantages and other advantages, and features of the present description will be readily apparent from the following Detailed Description when taken alone or in connection with the accompanying drawings. It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE DRAWINGS

Various aspects of this disclosure may be better understood upon reading the following detailed description and upon reference to the drawings in which:
FIG. 1 shows a block schematic diagram of an exemplary network of imaging system scanners, in accordance with one or more embodiments of the present disclosure;
FIG. 2 shows a block schematic diagram of an exemplary CT imaging system, in accordance with one or more embodiments of the present disclosure;
FIG. 3 shows a schematic diagram of a resource allocation system for allocating computing resources within a healthcare system, in accordance with one or more embodiments of the present disclosure;
FIG. 4 is a flowchart illustrating an exemplary high-level method for performing a medical imaging scan, where data processing tasks of the scan may be performed on various local and remote compute instances, in accordance with one or more embodiments of the present disclosure;
FIG. 5 is a flowchart illustrating a first exemplary method for assigning a compute instance for performing a data processing task of a medical imaging scan, in accordance with one or more embodiments of the present disclosure;
FIG. 6 is a flowchart illustrating a second exemplary method for assigning a compute instance for performing a data processing task of a medical imaging scan, in accordance with one or more embodiments of the present disclosure;
FIG. 7 is a flowchart illustrating an exemplary method for dynamically reassigning processing tasks based on data transmission latencies, in accordance with one or more embodiments of the present disclosure;
FIG. 8 is an exemplary reference table illustrating a model for determining a preference for local performance of a processing task, in accordance with one or more embodiments of the present disclosure;
FIG. 9 is an exemplary reference table showing a consumption of bandwidth and processing resources by various processing tasks, in accordance with one or more embodiments of the present disclosure; and
FIG. 10 is a schematic diagram showing a distribution of data processing tasks across various local and remote compute instances, in accordance with one or more embodiments of the present disclosure.

The drawings illustrate specific aspects of the described systems and methods. Together with the following description, the drawings demonstrate and explain the structures, methods, and principles described herein. In the drawings, the size of components may be exaggerated or otherwise modified for clarity. Well-known structures, materials, or operations are not shown or described in detail to avoid obscuring aspects of the described components, systems and methods.

### DETAILED DESCRIPTION

This description and embodiments of the subject matter disclosed herein relate to methods and systems for medical imaging systems that generate three dimensional (3D) image volumes of scanned object, such as an anatomy of a patient. While a computed tomography imaging (CT) system is described herein, it should be appreciated that the systems and methods described herein may be used with other types of medical imaging systems without departing from the scope of this disclosure. For example, the systems and methods may be used with a magnetic resonance (MR) imaging system, a positron emission tomography (PET) system, a dual-energy CT scanner, a photon-counting CT scanner, or a different kind of imaging system.

As the amount of signal processing performed by CT, MR, PET, and other medical imaging systems increases, processing the data acquired by such systems to generate images is increasingly performed via general purpose CPU-based and GPU-based infrastructure rather than specialty FPGAs, DSPs and microcontrollers. Using this general purpose computing infrastructure for processing the acquired data may facilitate reuse of computational infrastructure for various purposes. This reuse may be facilitated (a) within a particular apparatus, such as a scanner; (b) between like apparatuses (e.g., two CT machines); (c) between unlike imaging apparatuses (e.g., between a CT machine and an MR machine); (d) between arbitrary apparatuses (e.g., a CT machine and an AI model for automated diagnostics); and/or (e) between a local apparatus and a remote apparatus (e.g., a CT machine and a cloud application for PET reconstruction).

A computational infrastructure, or compute instance (CI), associated with a particular scanner or associated local device is referred to herein as local compute instance (LCI) (e.g., local to the scanner). The LCI may control a scanner, perform signal conditioning, and process sensor input. Using the LCI for such tasks, as opposed to a remote CI, such as a cloud compute instance (CCI), may minimize temporal latency and ensure stable real-time or near-real-time processing.

However, for some scanners, such as CT scanners, each scan may last only a few seconds. In many cases, the LCI may be used for such low latency applications for a portion (e.g., a small fraction) of a CT scanners total time available (e.g., a low duty cycle). As a consequence, after a scan, the LCI may be repurposed for other data processing applications, such as tomographic image reconstruction, image post-processing, etc. The repurposing of the LCI may include processing data on a same scanner; processing of data acquired by another CT scanner; and/or processing of data acquired by a different type of scanner (e.g., where an MR scanner uses a CT LCI for image reconstruction, etc.). Additionally or alternatively, the LCI may be advantageously used for performing artificial intelligence (AI) related tasks, such as automated opportunistic screening for health conditions by applying an AI detection model to an institution's database of images, participating in a multi-site federated learning scheme to train an AI model, and/or others.

However, management of LCI resources, including determining which tasks are allocated to a certain LCI and when they should be allocated, may not be performed efficiently. As a result, LCI resources may be available and unused, while other more costly or less efficient resources are used to perform processing tasks. For example, a data processing task may be performed by processing resources on a cloud-based server when an LCI resource is available, where processing the data on the cloud may be more time consuming and less efficient than using the LCI resource. Additionally, cloud hardware may be expensive to rent, and dispatching tasks to a number of local processing units may allow for more cost-effective operation.

To address this problem, systems and methods are disclosed herein for increasing an efficiency of use of LCI resources, based on adaptive prioritization of tasks within and between LCIs and centralized task assignment. As noted above, it is often preferred that the LCI be dedicated to latency-sensitive tasks during a scan, where when operating in standalone mode, the LCI will perform a certain base set of operations according to its design. When a scan is not being performed, the LCI may be operated in a cooperative mode, where the LCI may be assigned and/or accept computational tasks. Such tasks may be containerized. As opposed to other systems where requests for service may be sent to compute instances to determine availability, task assignment herein may be performed via a centralized controller, such as a resource allocation system, which may temporarily defer, prioritize, or deprioritize non-local tasks without sending requests for service and receiving responses. As a result, an effective boundary defining where processing is done (e.g., between local resources, and between local and external computation resources) may be adjusted in real time, where LCI/CI allocation may be performed via a dynamic descending priority list. In this way, image processing tasks may be advantageously distributed across various LCIs to increase an overall efficiency of use of LCI resources, increase imaging system uptime, and/or to minimize a time taken for image reconstruction.

In particular, the methods and systems described herein may be advantageously used to increase an efficiency with which AI models relied on by an imaging system may be refined or retrained. Various AI models may be deployed for use in different data processing tasks, which may undergo periodic or constant refinement. While there are advantages to operating such AI models using resources located on the cloud (e.g., remote resources), since medical scanners may have to be operable even if external network connections fail, fallback operation using an LCI (local to a machine or facility) is highly desirable. For such purpose, local versions of such models may be cached, for operation on an LCI. A failure of external network connections may generate a redistribution of assignment of tasks to one or more LCIs within a still-connected network of resources. For example, a multisite hospital with operable intranet connections may redistribute tasks to resources that remain available.

Similarly, in the event of the failure of an LCI resource, tasks assigned to the LCI resource may be reassigned to alternative LCLs and/or cloud-based resources. For example, in the event of failure of an image reconstruction server, images from the scanner may be reconstructed using a different available resource.

Further, in some embodiments, a computation accounting system may be implemented, whereby external use of an LCI may be offered in exchange for computation and/or monetary compensation to obtain efficiency or financial goals. Such paid resource-sharing may be performed between departments of a single institution, or between different entities. In some examples, LCI capabilities within an apparatus may be owned or leased by a different entity to the owner of the apparatus. An amount of compensation may be based on not just on the actual computation performed, but may also be adjusted based on task priority assigned, execution speed desired, and other factors such as electricity (or another utility) cost at the time the computation is performed. Thus, the assignment of resources across a network containing LCIs may be determined in part by the cost, capability, and availability of distributed local resources, on an adaptive, time-varying basis. As a result, an overall availability of scanner units may be increased, while reducing a processing time and a cost of data processing tasks.

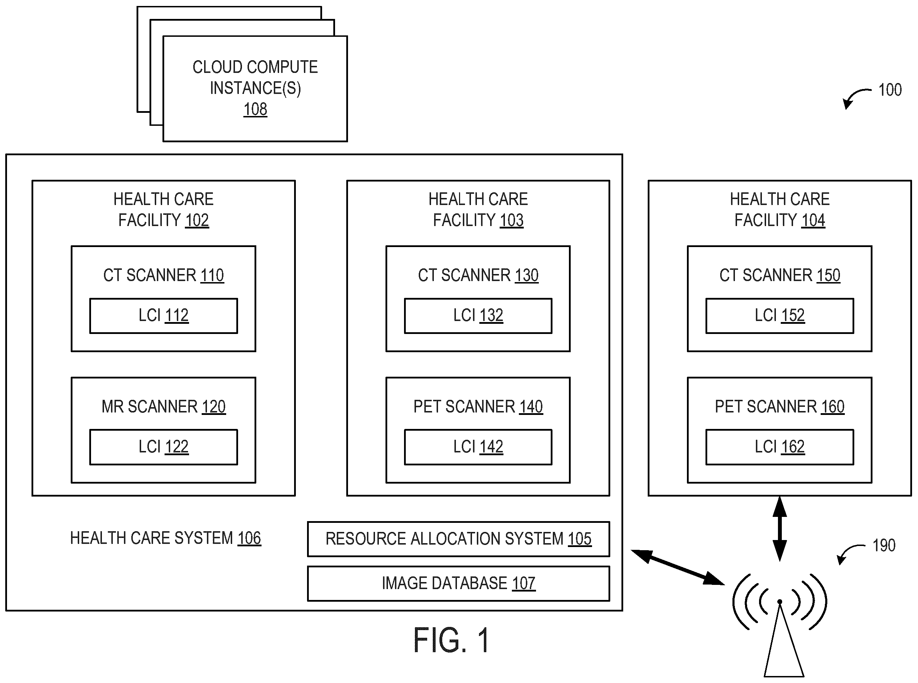

Referring now to the figures, FIG. 1 shows an exemplary simplified medical imaging ecosystem 100 including various networked health care facilities having imaging systems. Specifically, medical imaging ecosystem 100 includes a first healthcare facility 102 and a second healthcare facility 103, which are operated as part of a health care system 106. That is, healthcare facilities 102 and 103 may be managed by a same administration, and may be electronically connected to a same internal network of health care system 106.

Health care facility 102 includes a CT scanner 110 and an MR scanner 120. CT scanner 110 includes an LCI 112, which may be used to process projection data acquired via CT scanner 110 to reconstruct a CT image. Similarly, MR scanner 120 includes an LCI 122, which may be used to process projection data acquired via MR scanner 120 to reconstruct an MR image.

Health care facility 103 includes a CT scanner 130 and a PET scanner 140. CT scanner 130 includes an LCI 132, which may be used to process projection data acquired via CT scanner 130 to reconstruct a CT image. Similarly, PET scanner 140 includes an LCI 142, which may be used to process projection data acquired via PET scanner 140 to reconstruct a PET image.

However, within health care system 106, CT scanner 110, MR scanner 120, CT scanner 130, and PET scanner 140 may not all be in use at the same time. For example, on a first day, a demand for CT scanner 110 and CT scanner 130 may be high, and LCIs 112 and 132 may be in continuous use, while a demand for MR scanner 120 and PET scanner 140 may be lower, and there may be periods of time on the first day during which LCIs 122 and 142 may not be in use. On a second day, a demand for CT scanner 110 and CT scanner 130 may be lower, and LCIs 112 and 132 may not be in continuous use, while a demand for MR scanner 120 and PET scanner 140 may be higher, and LCIs 122 and 142 may be in continuous use.

To increase an efficiency of use of LCIs 112, 122, 132, and 142, health care system 106 includes a resource allocation system 105, which may be used to advantageously distribute processing tasks across LCIs 112, 122, 132, and 142 in a balanced manner. For example, on the first day, resource allocation system 105 may distribute some of the processing tasks typically performed on LCI 112 and LCI 132 to one or both of LCI 122 and LCI 142. For example, a scan may be performed on CT scanner 110, and projection data may be acquired during the scan. However, rather than processing the projection data on LCI 112 to reconstruct an image from the projection data, the projection data may be transmitted to LCI 142, and the projection data may be processed on LCI 142 to reconstruct the image. The reconstructed image data may then be stored in an image database 107 of health care system 106, where image database 107 may be accessible by radiologists and caregivers of both of health care facility 102 and health care facility 104.

By performing the image reconstruction on LCI 142 rather than LCI 112, a time taken by LCI 112 to process the CT scan may be reduced. As a result of the reduction in time spent by LCI 112 on the CT scan, an availability of CT scanner 110 for use on other patients may be increased, whereby a number of CT scans may be performed using CT scanner 110 may be increased. In this way, the demand on CT scanner 110 may be advantageously met/reduced by performing the image reconstruction on LCI 142. The processing on LCI 142 may be performed at a time when LCI 142 is not being used, as a result of the lower demand on PET scanner 140. On the second day, when MR scanner 120 and PET scanner 140 are in high demand, processing tasks typically performed via LCI 122 and LCI 142, respectively, may be similarly performed on one or both of LCI 112 and LCI 132, to increase a throughput of MR scanner 120 and PET scanner 140.

Medical imaging ecosystem 100 further includes a third health care facility 104, which includes a CT scanner 150 and a PET scanner 160. Data processing tasks performed on data acquired via CT scanner 150 may be performed on an LCI 152, and data processing tasks performed on data acquired via PET scanner 160 may be performed on an LCI 162. Health care facility 104 is not included within health care system 106. For example, health care facility 104 may be included within a different health care system. However, LCIs 152 and 162 may be communicatively coupled to health care system 106 via an external network 190. As such, resource allocation system 105 may access LCI 152 and/or LCI 162 via the external network 190. As a result, when demand may be high at all of CT scanner 110, MR scanner 120, CT scanner 130, and PET scanner 140, and all of LCIs 112, 122, 132, and 142 may be in use, resource allocation system 105 may transmit processing tasks to health care facility 104 to be performed on LCI 152 and/or LCI 162 when LCI 152 and/or LCI 162 are not being used, in the manner described above.

That is, in some embodiments, health care facility 104 may offer data processing as a service on LCI 152 and/or LCI 162 when LCI 152 and/or LCI 162 are not being used. Health care system 106 may compensate health care facility 104 via a financial arrangement, which may benefit both health care system 106 (e.g., increased throughput) and health care facility 104 (e.g., additional income). Conversely, health care system 106 may offer data processing as a service on one or more of LCIs 112, 122, 132, and 142 when LCIs 112, 122, 132, and 142 are not in use.

Medical imaging ecosystem 100 also includes one or more cloud compute instances (CCIs) 108, which may be communicatively coupled to health care facility 102, health care facility 103, and/or health care facility 104 via the Internet. As a result, resource allocation system 105 may also avail of CCIs 108 for performing data processing tasks that would typically be performed on one or more of LCIs 112, 122, 132, and 142. For example, if all of LCIs 112, 122, 132, and 142 are in use and demand is high for one or more scanners of health care facility 102 and health care facility 103, resource allocation system 105 may advantageously transmit a portion of the data processing tasks that would typically be performed on one or more of LCIs 112, 122, 132, and 142 to one or more CCIs 108. Additionally, some processing tasks may rely on greater processing and memory resources than may be available on LCIs 112, 122, 132, and 142.

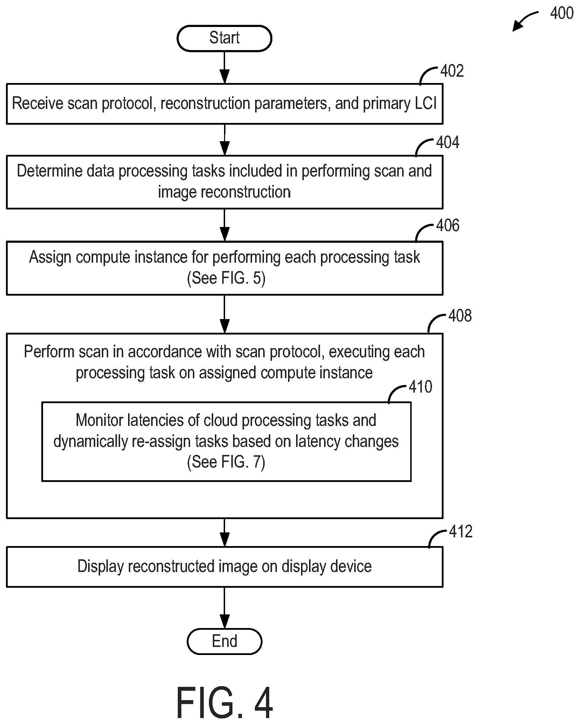

For such purpose, resource allocation system 105 may monitor availability of LCIs 112, 122, 132, and 142 of health care system 106, availability of LCIs 152 and 162 of health care facility 104, and data transmission latencies between health care system 106 and CCIs 108 to determine strategies for distributing processing tasks throughout medical imaging ecosystem 100 in a manner that maximizes an efficiency of use of CT scanner 110, MR scanner 120, CT scanner 130, and PET scanner 140, and balances workloads between LCIs 112, 122, 132, and 142. The strategies may include dynamically prioritizing and reprioritizing tasks in accordance with availability and latency data, as described in greater detail below in reference to FIGS. 4-10.

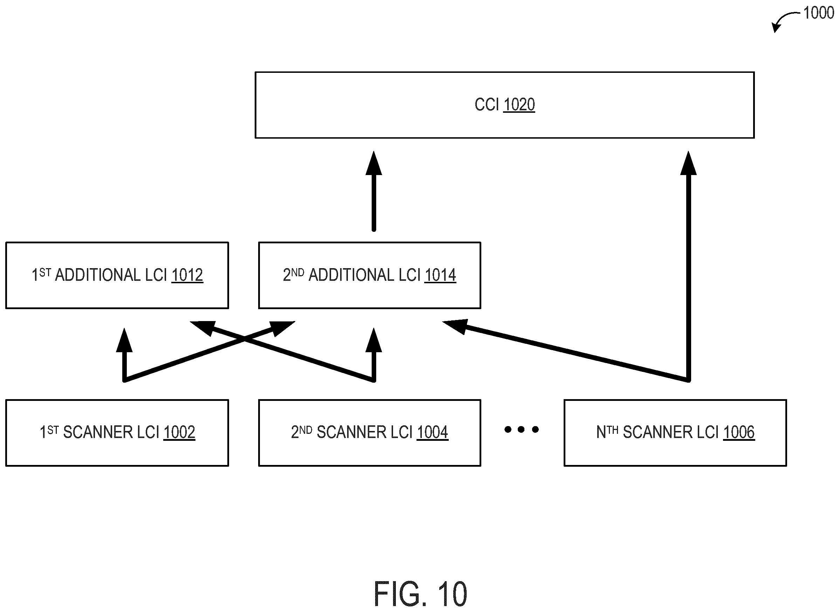

The strategies may also include following a hierarchy of CIs, where processing tasks may first be first assigned to a primary LCI, where the primary LCI is an LCI integrated into or associated with a scanner, and subsequently reassigned to more remote CIs based on the strategies. For example, referring to FIG. 10, a medical imaging ecosystem 1000 such as medical imaging ecosystem 100 may include a plurality of scanner LCIs, such as a first scanner LCI 1002, a second scanner LCI 1004, and so on up to an N^{th} scanner LCI 1006. In one embodiment, processing tasks may first be assigned to a same scanner, meaning, when a scan is performed using first scanner LCI 1002, data acquired during the scan may be processed preferentially on first scanner LCI 1002; when a scan is performed using second scanner LCI 1004, data acquired during the scan may be processed preferentially on first scanner LCI 1004; and so on.

Additional LCIs may be communicatively coupled to first scanner LCI 1002, second scanner LCI 1004, and N^{th} scanner LCI 1006, as described above. For instance, a first additional LCI 1012 may be communicatively coupled to first scanner LCI 1002 and second scanner LCI 1004, and a second additional LCI 1014 may be communicatively coupled to first scanner LCI 1002, second scanner LCI 1004, and N^{th} scanner LCI 1006. Thus, to reduce a computational load on first scanner LCI 1002, one or more processing tasks typically performed at first scanner LCI 1002 may be transferred to and executed at one or both of first additional LCI 1012 and second additional LCI 1014. When the one or more processing tasks are finished, resulting images and or data may be transferred back to first scanner LCI 1002. Executing a processing task at first additional LCI 1012 or second additional LCI 1014 rather than first scanner LCI 1002 may be advantageous if an amount of time taken to transfer a first set of data of the processing task to first additional LCI 1012 or second additional LCI 1014, process the first set of data, and transfer a second set of data resulting from executing the processing task back to first scanner LCI 1002 is less than an amount of time taken to execute the processing task at first scanner LCI 1002. For example, first additional LCI 1012 and second additional LCI 1014 may have greater processing resources than first scanner LCI 1002. Executing the processing task at first additional LCI 1012 or second additional LCI 1014 rather than first scanner LCI 1002 may also be advantageous if it facilitates first scanner LCI 1002 being used to perform a greater number of scans during an operational shift, for example.

Medical imaging ecosystem 1000 may also include one or more CCIs 1020, which may be communicatively coupled to all of first additional LCI 1012, second additional LCI 1014, first scanner LCI 1002, second scanner LCI 1004, and N^{th} scanner LCI 1006. If during executing the processing task at first additional LCI 1012 or second additional LCI 1014 it is determined that an overall processing time (e.g., including data transmission times) of the first set of data may be reduced by executing the processing task at a CCI 1020, the processing task may be transferred to the CCI 1020 for processing. After the CCI 1020 has finished processing the processing task, the second set of data may be transmitted back to first scanner LCI 1002.

In other words, a processing task may be preferentially first reassigned to an additional LCI available via an internal or external network based on a first assessment of available resources of the additional LCI, and subsequently reassigned to a CCI based on a second assessment of available resources of the CCI. If no additional LCIs are available for reassignment of the processing task, then the processing task may be reassigned to the CCI based on the second assessment. By reassigning processing tasks in this manner, a general preference for performing processing tasks locally may be enforced. Performing the processing tasks locally may increase a stability and reliability of an image reconstruction process.

It should be appreciated that the examples shown in FIGS. 1 and 10 are for illustrative purposes, and in other examples, medical imaging ecosystem 100 may include scanners and LCIs of different types, distributed across different health care systems.

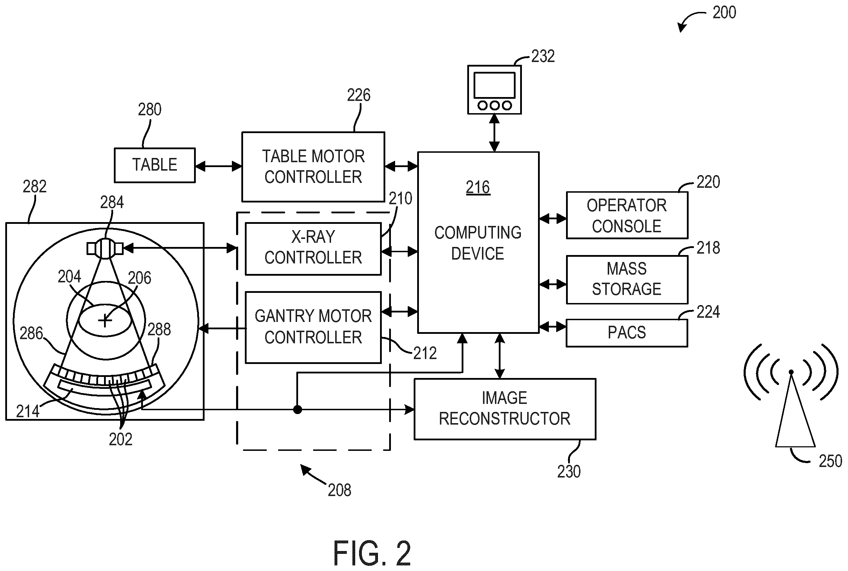

Referring now to FIG. 2, an exemplary X-ray imaging system 200 is shown, such as a CT imaging system. The X-ray imaging system 200 may be configured to image a subject laying on a table 280, such as a patient, an inanimate object, one or more manufactured parts, and/or foreign objects such as dental implants, stents, and/or contrast agents present within a body of the patient.

In some embodiments, the X-ray imaging system 200 includes a gantry 282, which in turn, may further include at least one X-ray source 284 configured to project a beam of X-ray radiation 286 for use in imaging the subject. Specifically, the X-ray source 284 is configured to project the X-ray radiation beams 286 towards a detector array 288 positioned on the opposite side of the gantry 282. The X-ray source 284 may project a cone-shaped X-ray radiation beam which is collimated to lie within an X-Y-Z plane of a Cartesian coordinate system and generally referred to as an "imaging plane." The X-ray radiation beam passes through an object being imaged, such as the subject. The X-ray radiation beam, after being attenuated by the object, impinges upon an array of detector elements 202 of a detector array 288. In some embodiments, detector array 288 may be fabricated in a multi-slice configuration including the plurality of detector elements 202, where one or more additional rows of the detector elements 202 are arranged in a parallel configuration for acquiring the projection data.

The intensity of the attenuated X-ray radiation beam received at the detector array is dependent upon the attenuation of an X-ray radiation beam by the object. Each detector element 202 of the array produces a separate electrical signal that is a measurement of the X-ray beam attenuation at the detector location. The attenuation measurements from all the detector elements are acquired separately to produce a transmission profile. A group of X-ray radiation attenuation measurements, e.g., projection data, from the detector array at one gantry angle is referred to as a "view." A "scan" of the object includes a set of views made at different gantry angles, or view angles, during one revolution of the X-ray source and detector.

The X-ray source 284 includes an anode and a cathode. Electrons emitted by the cathode (e.g., resulting from energization of the cathode) may be intercepted by a target arranged at or near the anode. Electrons intercepted by the target may release energy in the form of X-rays, with the X-rays being directed toward the detector array 288. An area of the target surface that receives the electrons from the cathode and forms the emitted X-rays may be referred to herein as a focal spot. The emitted X-rays may be focused on a portion of the subject, at an effective focal spot.

Although FIG. 2 depicts a single X-ray source 284, in certain embodiments, multiple X-ray sources and detectors may be employed to project a plurality of X-ray radiation beams for acquiring projection data at different energy levels corresponding to the patient. In some embodiments, the X-ray detector employed is a photon-counting detector which is capable of differentiating X-ray photons of different energies. In other embodiments, two sets of X-ray sources and detectors are used to generate dual-energy projections, with one set at low-kVp and the other at high-kVp.

In certain embodiments, the X-ray imaging system 200 is configured to traverse different angular positions around the subject 204 for acquiring desired projection data. Accordingly, the gantry 282 and the components mounted thereon may be configured to rotate about a center of rotation 206 for acquiring the projection data, for example, at different energy levels. Alternatively, in embodiments where a projection angle relative to the subject 204 varies as a function of time, the mounted components may be configured to move along a general curve rather than along a segment of a circle.

As the X-ray source 284 and the detector array 288 rotate, the detector array 288 collects data of the attenuated X-ray beams. The data collected by the detector array 288 undergoes pre-processing and calibration to condition the data to represent the line integrals of the attenuation coefficients of the scanned subject 204. The processed data are commonly called projections. In some examples, the individual detectors or detector elements 202 of the detector array 288 may include photon-counting detectors which register the interactions of individual photons into one or more energy bins. It should be appreciated that the methods described herein may also be implemented with energy-integrating detectors.

In one embodiment, the X-ray imaging system 200 includes a control mechanism 208 to control movement of the components such as rotation of the gantry 282 and the operation of the X-ray source 284. In certain embodiments, the control mechanism 208 further includes an X-ray controller 210 configured to provide power and timing signals to the X-ray source 284. Additionally, the control mechanism 208 includes a gantry motor controller 212 configured to control a rotational speed and/or position of the gantry 282 based on imaging requirements.

In certain embodiments, the control mechanism 208 further includes a data acquisition system (DAS) 214 configured to sample analog data received from the detector elements 202 and convert the analog data to digital signals for subsequent processing. The DAS 214 may be further configured to selectively aggregate analog data from a subset of the detector elements 202 into so-called macro-detectors, as described further herein. The data sampled and digitized by the DAS 214 is transmitted to a computer or computing device 216. In one example, the computing device 216 stores the data in a storage device or mass storage device 218. The storage device 218, for example, may be any type of non-transitory memory and may include a hard disk drive, a floppy disk drive, a compact disk-read/write (CD-R/W) drive, a Digital Versatile Disc (DVD) drive, a flash drive, and/or a solid-state storage drive.

Additionally, the computing device 216 provides commands and parameters to one or more of the DAS 214, the X-ray controller 210, and the gantry motor controller 212 for controlling system operations such as data acquisition and/or processing. The computing device 216 may act as an image processor unit configured to reconstruct images of a target volume of the subject using an iterative or analytic image reconstruction method. For example, the image processor unit may use an analytic image reconstruction approach such as filtered back projection (FBP) to reconstruct images of a target volume of the patient. As another example, the image processor unit may use an iterative image reconstruction approach such as advanced statistical iterative reconstruction (ASIR), conjugate gradient (CG), maximum likelihood expectation maximization (MLEM), model-based iterative reconstruction (MBIR), and so on to reconstruct images of a target volume of the subject.

In certain embodiments, the computing device 216 controls system operations based on operator input. The computing device 216 receives the operator input, for example, including commands and/or scanning parameters via an operator console 220 operatively coupled to the computing device 216. The operator console 220 may include a keyboard (not shown) or a touchscreen to allow the operator to specify the commands and/or scanning parameters.

Although FIG. 2 illustrates one operator console 220, more than one operator console may be coupled to the X-ray imaging system 200, for example, for inputting or outputting system parameters, requesting examinations, plotting data, and/or viewing images. Further, in certain embodiments, the X-ray imaging system 200 may be coupled to multiple displays, printers, workstations, and/or similar devices located either locally or remotely, for example, within an institution or hospital, or in an entirely different location via one or more configurable wired and/or wireless networks such as the Internet and/or virtual private networks, wireless telephone networks, wireless local area networks, wired local area networks, wireless wide area networks, wired wide area networks, etc.

In one embodiment, for example, the X-ray imaging system 200 either includes, or is coupled to, a picture archiving and communications system (PACS) 224. In an exemplary implementation, the PACS 224 is further coupled to a remote system such as a radiology department information system, hospital information system, and/or to an internal or external network (not shown) to allow operators at different locations to supply commands and parameters and/or gain access to the image data.

The computing device 216 uses the operator-supplied and/or system-defined commands and parameters to operate a table motor controller 226, which in turn, may control a table 114 which may be a motorized table. Specifically, the table motor controller 226 may move the table 114 for appropriately positioning the subject 204 in the gantry 282 for acquiring projection data corresponding to the target volume of the subject 204.

As previously noted, the DAS 214 samples and digitizes the projection data acquired by the detector elements 202. Subsequently, an image reconstructor 230 uses the sampled and digitized X-ray data to perform high-speed reconstruction. Although FIG. 2 illustrates the image reconstructor 230 as a separate entity, in certain embodiments, the image reconstructor 230 may form part of the computing device 216. Alternatively, the image reconstructor 230 may be absent from the X-ray imaging system 200 and instead the computing device 216 may perform one or more functions of the image reconstructor 230. Moreover, the image reconstructor 230 may be located locally or remotely, and may be operatively connected to the X-ray imaging system 200 using a wired or wireless network. Particularly, one exemplary embodiment may use computing resources in a "cloud" network cluster for the image reconstructor 230.

In one embodiment, the image reconstructor 230 stores the images reconstructed in the storage device 218. Alternatively, the image reconstructor 230 may transmit the reconstructed images to the computing device 216 for generating useful patient information for diagnosis and evaluation. In certain embodiments, the computing device 216 may transmit the reconstructed images and/or the patient information to a display or display device 232 communicatively coupled to the computing device 216 and/or the image reconstructor 230. In some embodiments, the reconstructed images may be transmitted from the computing device 216 or the image reconstructor 230 to the storage device 218 for short-term or long-term storage.

Additionally, either or both of computing device 216 and image reconstructor 230 may be communicatively coupled to a network 250, such as a wireless network. Via the network 250, computing device 216 and/or image reconstructor 230 may be in communication with other computing devices of other imaging systems, and/or cloud-based CCIs. The other imaging systems may include imaging systems within a same health care facility, or a different health care facility, including outside of a health care system of X-ray imaging system 200. Via the network 250, processing tasks typically performed on computing device 216 and image reconstructor 230 may be sent to the other computing devices (e.g., LCIs) of other imaging systems and/or the cloud-based CCIs for processing, as described above in reference to FIG. 1. Processing tasks may also be received from the other computing devices and performed on computing device 216 and/or image reconstructor 230.

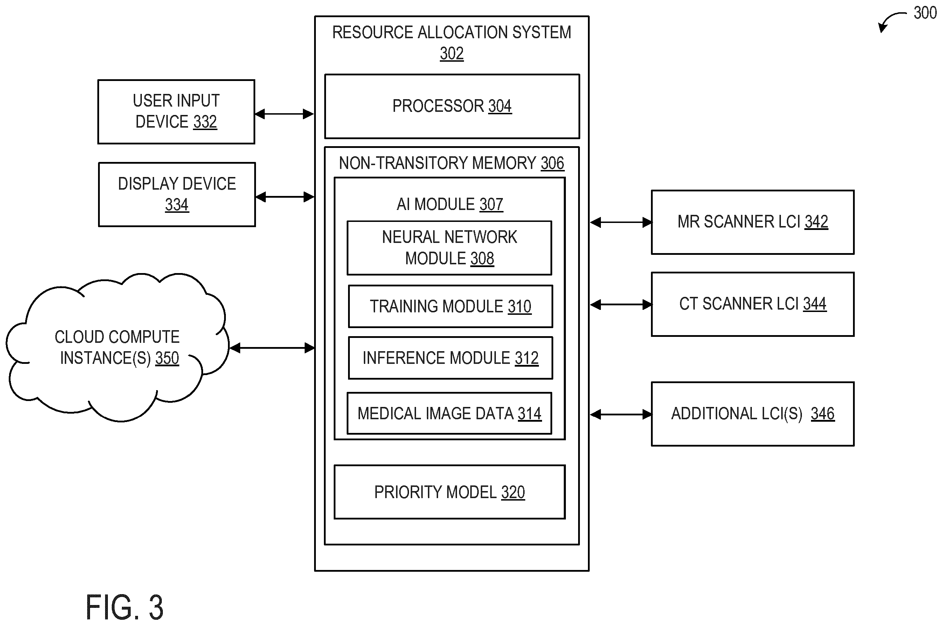

Referring now to FIG. 3, an exemplary resource allocation system 302 of a medical imaging ecosystem 300 is shown, where medical imaging ecosystem 300 may be a non-limiting example of medical imaging ecosystem 100 of FIG. 1. Medical imaging ecosystem 300 may include one or more imaging systems, such as a CT imaging system (e.g., X-ray imaging system 200 of FIG. 2), an MR imaging system, a PET imaging system, or a different kind of imaging system, which may be communicatively coupled to each other and to resource allocation system 302. In particular, resource allocation system 302 may be communicatively coupled to an MR scanner LCI 342, a CT scanner LCI 344, and one or more additional LCIs of additional imaging systems (e.g., other CT imaging systems, other MR imaging systems, a PET imaging system, a SPECT imaging system, a CT-GSI imaging system, and/or a different type of imaging system).

In various examples, imaging data from MR scanner LCI 342, CT scanner LCI 344, and the one or more additional LCIs of additional imaging systems may be received and processed via processor 304 based on instructions stored in one or more modules of non-transitory memory 306, and/or stored in non-transitory memory 306 (e.g., medical image data 314).

Resource allocation system 302 may be used to allocate resources within medical imaging ecosystem 300, as described in greater detail herein. Resource allocation system 302 may be disposed at a computing device local to one or more of the one or more imaging systems of medical imaging ecosystem 300, and may be communicably coupled to local and remote elements of the medical imaging ecosystem 300 via wired and/or wireless connections. The computing device may constitute a local compute instance (LCI) available for performing computational tasks inherent to a functioning of the one or more imaging systems, including image processing tasks such as image segmentation, pathology detection, noise/artifact removal, etc. Medical imaging ecosystem 300 may also include one or more cloud compute instances (CCIs) 350, which may be communicatively coupled to resource allocation system 302 and may comprise additional processing resources available for performing the computational tasks.

Processor 304 may be configured to execute machine readable instructions stored in non-transitory memory 306. Processor 304 may be single core or multi-core, and the programs executed thereon may be configured for parallel or distributed processing. In some embodiments, the processor 304 may optionally include individual components that are distributed throughout two or more devices, which may be remotely located and/or configured for coordinated processing. In some embodiments, one or more aspects of the processor 304 may be virtualized and executed by remotely-accessible networked computing devices configured in a cloud computing configuration.

Non-transitory memory 306 may store an AI module 307, which may include various AI models and algorithms that may be applied to image data received from the one or more imaging systems and/or other elements of imaging ecosystem 300. The various AI models may include probabilistic models, statistical models, rules-based models, as well as machine learning (ML) and/or deep learning (DL) neural network models, and instructions for implementing the AI, ML and/or DL models to perform various tasks on medical images generated by imaging ecosystem 300. For example, among other things, the AI models may be used to detect, identify, segment, label, and/or extract features of the medical images, including anatomical features (e.g., organs, systems, vessels, arteries, bones, etc.) and findings (e.g., tumors, nodules, lesions, scarring, etc.). In some examples, AI module 307 includes instructions for implementing one or more gradient descent algorithms, applying one or more loss functions, and/or training routines, for use in adjusting parameters of the ML and/or DL models. In some examples, AI module 307 includes instructions for training the ML and/or DL using federated learning, where training data is received from the one or more imaging systems in a distributed manner.

Specifically, AI module 307 may store a neural network module 308, a network training module 310, an inference module 312, and medical image data 314. Neural network module 308 may include one or more of the ML and/or DL models and instructions for implementing the of the ML and/or DL models to perform the various tasks. Neural network module 308 may include one or more trained and/or untrained neural networks and may further include various data, or metadata pertaining to the one or more neural networks stored therein.

Training module 310 may comprise instructions for training one or more of the neural networks implementing a DL model stored in neural network module 308. Inference module 312 may store one or more trained neural networks or other types of trained models for performing the various tasks.

Non-transitory memory 306 further stores medical image data 314. Medical image data 314 may include images acquired by the one or more imaging systems. Medical image data 314 may include for example, medical images acquired via a scanner, which may be an MRI scanner, a CT scanner, a scanner for spectral imaging, or via a different imaging modality of the imaging ecosystem 300. The scanner may be any imaging device configured to image a subject such as a patient, an inanimate object, one or more manufactured parts, and/or foreign objects such as dental implants, stents, and/or contrast agents present within the body. The medical images and imaging data stored as medical image data 314 may be processed based on instructions stored in non-transitory memory 306, and may be processed by one or more AI models stored in AI module 307.

Resource allocation system 302 may be operably/communicatively coupled to a user input device 332 and a display device 334. User input device 332 may comprise one or more of a touchscreen, a keyboard, a mouse, a trackpad, a motion sensing camera, or other device configured to enable a user to interact with and manipulate data within resource allocation system 302. Display device 334 may include one or more display devices utilizing virtually any type of technology. In some embodiments, display device 334 may comprise a computer monitor, and may display medical images. Display device 334 may be combined with processor 304, non-transitory memory 306, and/or user input device 332 in a shared enclosure, or may be peripheral display devices and may comprise a monitor, touchscreen, projector, or other display device known in the art, which may enable a user to view medical images produced by medical imaging ecosystem 300, and/or interact with various data stored in Medical image data 314 and non-transitory memory 306. In some examples, the display device 334 may be the same as or similar to display device 232 of FIG. 2.

Non-transitory memory 306 may also include a priority model 320. Resource allocation system 302 may allocate resources of medical imaging ecosystem 300 to perform various computational tasks performed during operation of the one or more imaging systems based on priority model 320. Priority model 320 may take as input one or more computational tasks to be performed, and may output priority scores for each computational task of the one or more computational tasks. The priority scores may indicate a priority for performing a respective computational task at local processing resources, such as at the processor 304 (e.g., the local LCI) as opposed to one or more of MR scanner LCI 342, CT scanner LCI 344, and/or the additional LCIs 346 of the one or more imaging systems or CCI 350. The resources of processor 304, MR scanner LCI 342, CT scanner LCI 344, and/or the additional LCIs 346, and the one or more CCIs 350 may be assigned in accordance with the assigned priority scores.

It should be understood that resource allocation system 302 shown in FIG. 1 is for illustration, not for limitation. Another appropriate image processing system may include more, fewer, or different components.

Referring now to FIG. 4, a method 400 is shown for performing a scan using a medical imaging system and reconstructing an image from data acquired during the scan, where data processing tasks of the scan may be performed in a distributed manner on various local and remote compute instances to maximize an efficiency of use of the local and remote resources, and to reduce an amount of time spent on the scan. Method 400 and other methods described herein may be executed by a processor of a resource allocation system associated with one or more medical imaging systems, such as processor 304 of resource allocation system 302 of FIG. 3.

Method 400 begins at 402, where method 400 includes receiving a scan protocol and additional instructions for performing the scan, including reconstruction parameters or other settings that may not be included in the protocol, and a primary LCI. The primary LCI may be an LCI that is integrated into a scanner of the imaging system, such as MR scanner LCI 342, CT scanner LCI 344 of FIG. 3, and LCIs 112, 122, 132, 142, 152, and 162 of FIG. 1. The primary LCI may be the LCI typically used, or preferentially used, for performing processing tasks included in performing a scan on the scanner. For example, if the scanner is not connected to a network, all of the processing tasks included in performing the scan may be performed by the primary LCI. In some examples, the protocol, scan settings, reconstruction parameters, and/or additional instructions may be selected, adjusted, or customized by a user of the medical imaging system.

At 404, method 400 includes determining a plurality of processing tasks included in performing the scan and subsequent image reconstruction. The processing tasks may include tasks involved in executing the scan, such as scan setup (e.g., determining scan parameters and scan protocol, patient positioning, scan timing, injection parameters, etc.), scan technique selection, and real-time scan control during the scan (e.g., performing a real-time feedback loop to adjust the scan parameters based on raw scan data, ECG data, respiratory phase, or other sensor data). The processing tasks may also include quality control and full quality image reconstruction/generation tasks, such as hardware calibration, pile-up correction and other data corrections, material decomposition, filtering, backprojection, tomographic reconstruction, etc. The image reconstruction tasks may also include image processing tasks for increasing an image quality of a reconstructed image, such as denoising, artifact removal, motion compensation, and the like. The processing tasks may also include image analysis tasks, such as the detection and characterization of tumors, lesions, nodules, and/or other abnormalities in an image, which may be performed using one or more AI or ML models, segmentation, dimension estimation, registration, and report generation. In general, the processing tasks may include any processing tasks performed as part of a scanning process to generate an image of a desired quality for diagnosing a patient. It should be appreciated that many of the above tasks could be AI-based or non-AI-based.

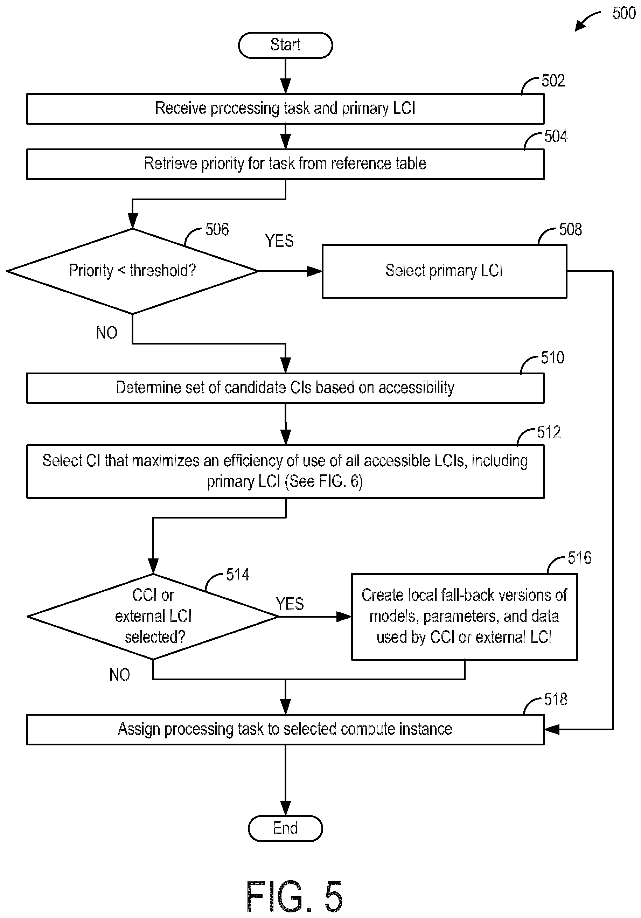

At 406, method 400 includes assigning a CI for performing each processing task of the plurality of processing tasks. Each processing task may be performed by a different CI, or various processing tasks may be performed by a single CI. The CI may be an LCI or a CCI. The CI may be assigned by following one or more steps of method 500 of FIG. 5, described below.

At 408, method 400 includes performing the scan in accordance with the scan protocol, executing each processing task on the assigned CI. For example, a first processing task or set of processing tasks of the scan may be performed on the primary LCI, such as the scan technique selection, the patient setup, and/or the real-time scan control. A second processing task or set of processing tasks of the scan may be performed on one or more LCIs of devices coupled to the primary LCI (e.g., LCIs 112, 122, 132, and 142 of FIG. 1), such as one or more image reconstruction tasks. A third processing task or set of processing tasks of the scan may be performed on one or more LCIs of devices connected to the primary LCI via an external network (e.g., LCIs 152 and 162), such as additional image processing tasks. A fourth processing task or set of processing tasks of the scan may be performed on one or more CCIs available via the Internet (e.g., CCIs 108), such as computationally intensive AI-based detection algorithms. Thus, each of the LCIs and CCIs may perform a portion of a total amount of processing involved in performing the scan. Each processing task may rely on input data, for example, from a previous processing task, and may produce output data, which may be an input to a subsequent processing task. In various embodiments, the output data may be stored in a memory of the resource allocation system, where the output data may be retrieved by one or more LCIs or CCIs for additional processing.

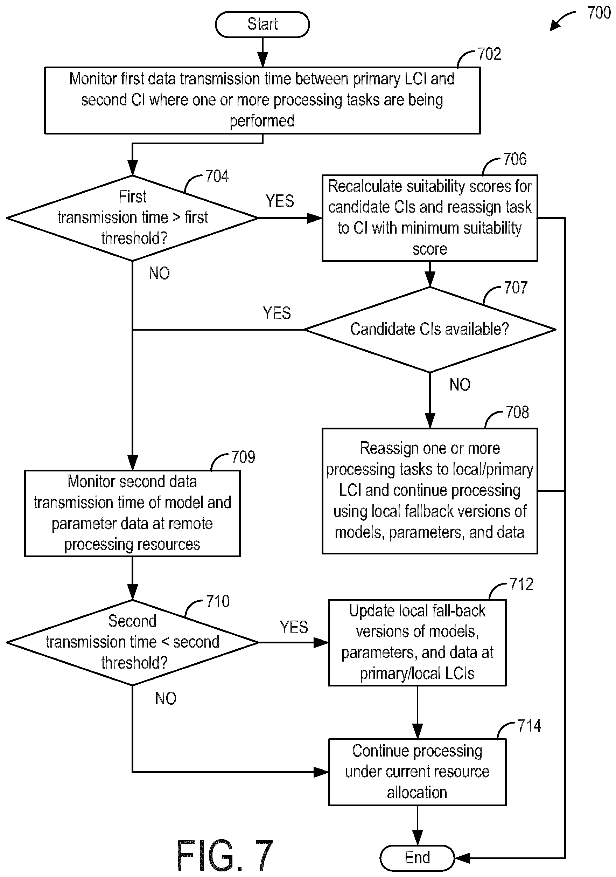

At 410, performing the scan in accordance with the scan protocol includes monitoring data transmission latencies when sending data between two or more LCIs and/or CCIs. If the data transmission latencies are above a threshold latency, one or more processing tasks may be reassigned to increase an overall efficiency of the imaging system, as described in greater detail below, in reference to FIG. 7.

At 412, method 400 includes displaying the reconstructed image on a display device, such as display device 334 of FIG. 3. Method 400 ends.

Referring now to FIG. 5, an exemplary method 500 is shown for assigning a CI for performing a data processing task of a medical imaging scan, in accordance with one or more embodiments of the present disclosure. In various embodiments, method 500 may be performed as part of method 400 of FIG. 4.

Method 500 begins at 502, where method 500 includes receiving a processing task to be performed, and a primary LCI associated with a scanner on which the scan was initiated. The processing task may be one of a plurality of processing tasks determined as a part of method 400.

At 504, method 500 includes retrieving a priority assigned to the task from a reference table. For example, the reference table may be stored in a memory of the resource allocation system (e.g., non-transitory memory 306). The reference table may indicate assigned priorities for a plurality of processing tasks. In various embodiments, the assigned priorities may be predefined manually by human experts, such as medical physicists or radiologists of a healthcare system of the medical imaging system (e.g., healthcare system 106). The priorities may be numeric values, such as numbers from 1 to 10. For example, the number one may indicate a highest priority; the number two may indicate a next highest priority: the number three may indicate a next highest priority; and so on. In some cases, different processing tasks may be assigned a same priority. The priority of the processing task may indicate a preferential order in which the processing task is performed. The priority may also indicate a preference for performing the processing task on local resources as opposed to remote resources, as described in greater detail below.

At 506, method 500 includes determining whether the assigned priority for the processing task retrieved from the reference table is less than a threshold priority. For example, the threshold priority may be three, where if the processing task is assigned a priority of one or two, the assigned priority is less than the threshold priority, and the answer is yes. Alternatively, if the assigned priority is a four, or five, or a higher number, the assigned priority is not less than the threshold priority, and the answer is no.

If at 506 it is determined that the priority assigned to the processing task is less than the threshold priority, method 500 proceeds to 508. At 508, method 500 includes selecting the primary LCI for performing the processing task, and at 518, assigning the processing task to the primary LCI. In other words, higher or highest-priority processing tasks may be assigned to an LCI integrated into the scanner used to perform the scan. In some cases, a processing task with a high priority may only be performed at the primary LCI. For example, some scan setup tasks may not be performed on a remote LCI or CCI.

Alternatively, if at 506 it is determined that the priority assigned to the processing task is not less than the threshold priority, method 500 proceeds to 510. At 510, method 500 includes determining a set of candidate CIs for performing the processing task, based on an accessibility of CIs coupled to or connected to the primary LCI and/or the resource allocation system. For example, the primary LCI may be integrated into a CT scanner of a healthcare facility (e.g., CT scanner 110 of healthcare system 106). The healthcare facility may include a different type of scanner, such as an MR scanner (e.g., MR scanner 120), which may have an LCI on which the processing task could be performed. A different scanner at a different healthcare facility (e.g., CT scanners 130 and 150, and pet scanners 140 and 160) may have an LCI on which the processing task could be performed, where the different healthcare facility may be a healthcare facility of a same healthcare system (e.g., healthcare facility 103), or a healthcare facility external to the healthcare system (e.g., healthcare facility 104). Any LCIs detected via local or external networks may be considered candidate CIs. In other words, an inventory of candidate CIs for performing the processing task may be generated, which may include the primary LCI and any accessible CCIs.

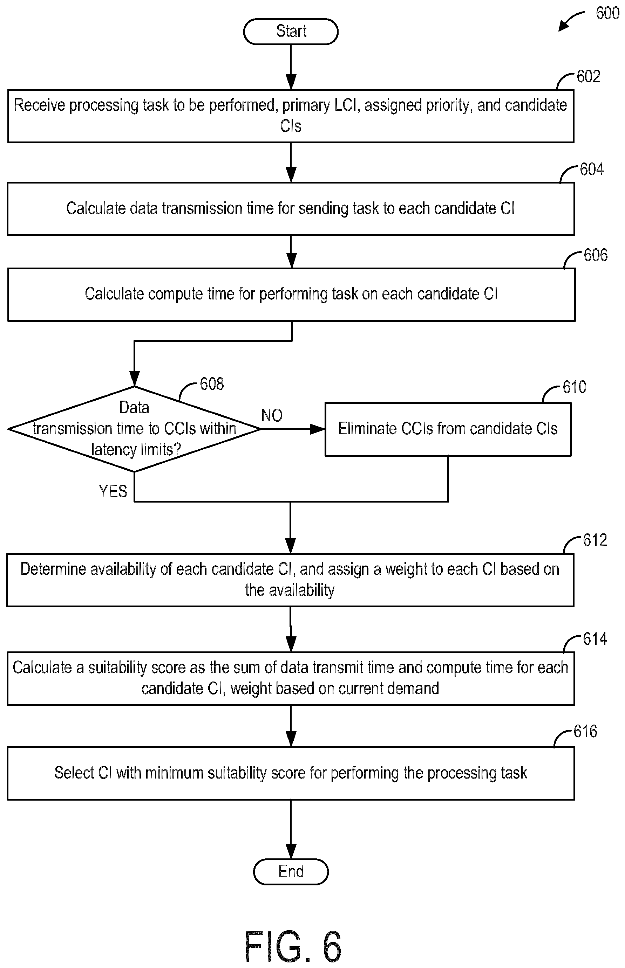

At 512, method 500 includes selecting a CI for performing the processing task that maximizes an efficiency of use of all candidate CIs, including the primary LCI, based on the assigned priority. Selecting the CI that maximizes the efficiency of use of all the candidate CIs is described below in reference to FIG. 6.

At 514, method 500 includes determining whether a CCI or an external LCI was selected for performing the processing task, where the CCI or external LCI relies on a network to transmit data to or from the primary LCI. If the processing task was assigned to a CCI or external LCI, method 500 proceeds to 516.

At 516, method 500 includes creating local fallback versions of models, parameters, and data used by the CCI to perform the processing task. Since medical scanners are relied on to be operable even if external network connections fail, a fallback operation using the primary LCI, or a different LCI within a same healthcare facility of the scanner/primary LCI, may be desirable. For such purpose, local versions of AI and/or ML models used by the CCI may be cashed at the primary LCI or the different LCI, such that a failure of external network connections may result in a redistribution of the assignment of the task to an LCI within a still-connected network of resources. For example, a multisite hospital may lose an Internet connection, but may redistribute tasks to local resources of the multi-site hospital that remain accessible. In such cases, cached versions of the AI and/or ML models may be used to resume execution of the processing task on the local resources.

At 518, method 500 includes assigning the processing task to the selected CI, and method 500 ends.

Referring now to FIG. 6, a method 600 is shown for selecting a CI that maximizes an efficiency of use of a plurality of candidate CIs, including a primary LCI, LCIs within a shared facility, LCIs outside the shared facility, and CCIs located in a cloud. In various embodiments, method 600 may be performed as part of method 500 of FIG. 5 described above. It should be appreciated that in different embodiments, one or more steps of method 600 may be performed in a different order than described below.

Method 600 begins at 602, where method 600 includes receiving a data processing task to be performed, a primary LCI where the task was generated, an assigned priority of the processing task, and a selection of candidate CIs, which may be determined in accordance with method 500. The processing task may be part of a process for reconstructing an image, or the processing task may be a post-reconstruction processing task. For example, the post-reconstruction task may include analyzing a reconstructed image to detect a health condition of a patient of the reconstructed image using an AI model.

At 604, method 600 includes calculating a data transmission time for transmitting the processing task to each candidate CI. The data transmission time may include a first amount of time during which the data is transmitted to the candidate CI, and a second amount of time during which data generated during processing of the processing task is transmitted back to the primary LCI from the candidate CI. In the event that the candidate CI is the primary LCI, the data transmission time may be negligible. The data transmission time may be calculated based on a size or amount of data to be transmitted and a current measured data transmission rate between the primary LCI and the candidate CI, based on available bandwidth at a time of assigning the processing task.

At 606, method 600 includes calculating a compute time for performing the task on each candidate CI. The compute time may be estimated based on historical processing times for similar processing tasks and available computational and memory resources at each available CI. In some embodiments, the compute times may be predefined by human experts and stored in a lookup table, and calculating the compute time may include retrieving the compute time from the lookup table.

At 608, method 600 includes determining whether the data transmission time to candidate CCIs are within acceptable latency limits for transmitting the processing task to the candidate CCIs. The acceptable latency limits may be different for processing tasks of different priorities. In various examples, a model, such as a decision tree model, may be used to determine whether the data transmission time to a candidate CCI is within acceptable latency limits for transmitting the processing task, based on the priority of the processing task.

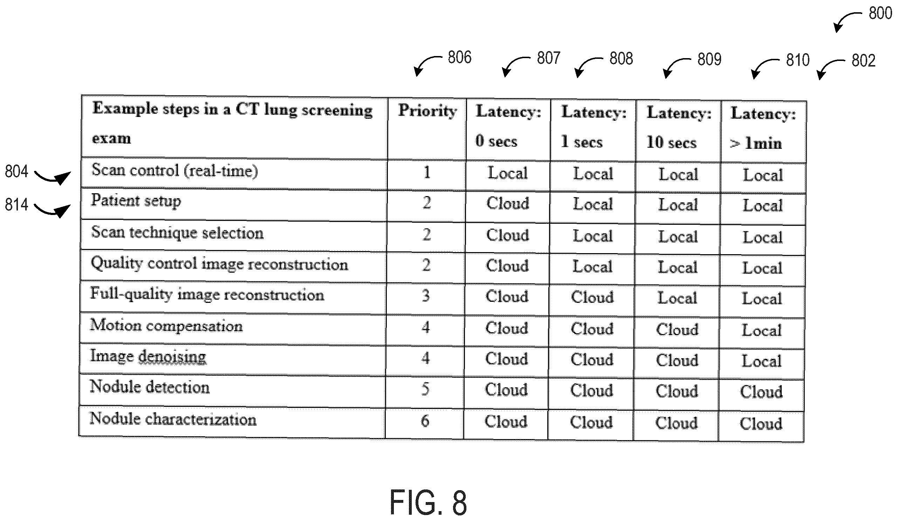

Referring briefly to FIG. 8, an exemplary model 800 is shown for determining whether a data transmission time is within acceptable latency limits for transmitting various processing tasks of a lung screening exam to a candidate CCI. Model 800 is a decision tree model depicted as a table 802, where rows of table 802 indicate processing tasks included in the lung screening exam, and latency conditions of model 800 are imposed by a series of columns of table 802. Thus, model 800 may be implemented (e.g., by a processor) by reading table 802, where a cell of table 802 indicates a preference between local processing and cloud-based processing for a task corresponding to a row of the cell, based on a latency limit indicated by a column of the cell. In some examples, a user is able to edit the table according to their preferences.

For example, a first processing task for real-time scan control is shown in a first row 804 of table 802. Reading first row 804 across table 802, a first column 806 indicates that the priority of the first processing task is 1. For the priority of 1, a second column 807 indicates that if the transmission time for sending data of the first processing task to and from the candidate CCI is negligible, then the first processing task is preferentially performed locally, meaning, at an LCI, and not a CCI. A third column 808 indicates that if the transmission time for sending data of the first processing task to and from the candidate CCI is greater than one second, then the first processing task is preferentially performed locally. A fourth column 809 indicates that if the transmission time for sending data of the first processing task to and from the candidate CCI is greater than 10 seconds, then the first processing task is preferentially performed locally. A fifth column 810 indicates that if the transmission time for sending data of the first processing task to and from the candidate CCI is greater than one minute, then the first processing task is preferentially performed locally.

A second processing task for patient setup is shown in a second row 814 of table 802. Reading second row 814 across table 802, first column 806 indicates that the priority of the first processing task is 2. For the priority of 2, second column 807 indicates that if the transmission time for sending data of the second processing task to and from the candidate CCI is negligible, then the second processing task may be performed on the CCI rather than locally. Third column 808 indicates that if the transmission time for sending data of the second processing task to and from the candidate CCI is greater than one second, then the second processing task is preferentially performed locally. Fourth column 809 indicates that if the transmission time for sending data of the second processing task to and from the candidate CCI is greater than 10 seconds, then the second processing task is preferentially performed locally. Fifth column 910 indicates that if the transmission time for sending data of the second processing task to and from the candidate CCI is greater than one minute, then the second processing task is preferentially performed locally.

In this way, table 802 indicates, for each processing task of the lung screening exam, whether the processing task is preferentially performed on an LCL, or whether the processing task could be performed on a CCI. Some low-priority processing tasks, such as the real-time scan control, may be preferentially performed on a primary LCL (e.g., where the exam is being performed) regardless of data transmission times to a CCI, for practical, logistical, or legal reasons. Such low-priority processing tasks may be addressed at step 508 of method 500. Other, higher-priority tasks may be performed either at an LCL or at a CCI, depending on the data transmission time to and from the CCI. Thus, model 800 may be used to broadly determine whether a CCI may be suitable for performing a processing task based on the data transmission time to and from the CCI.

The determination of the preference for performing a processing task at an LCL as opposed to performing the processing task on a CCI may also be based on the compute time for the processing task on the CCI. In some embodiments, a general preference for local vs. remote processing may initially be determined based on general estimations of available bandwidth and compute time that are predefined (for example, by human experts) and stored in a lookup table. The general preferences may then be broken down into specific preferences of local vs. remote processing in the model based on different latency thresholds for specific CCIs, as described above.

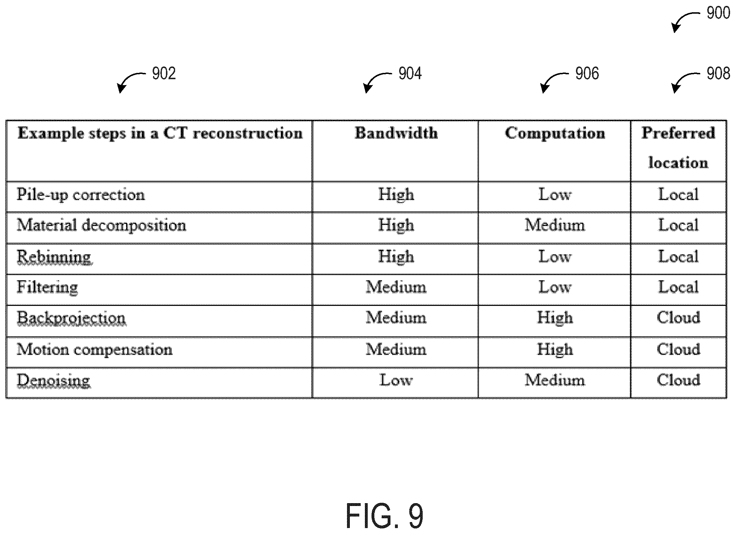

Referring briefly to FIG. 9, an exemplary lookup table 900 shows a set of general preferences for local vs. remote processing for a set of processing tasks of a CT image reconstruction. A first column 902 of lookup table 900 includes a listing of the set of processing tasks. A second column 904 includes, for each processing task of the set of processing tasks, an estimated amount of bandwidth relied on for transmitting data associated with performing the processing task to a generic CCI. A third column 906 includes, for each processing task of the set of processing tasks, an estimated amount of computation associated with performing the processing task on the generic CCI. A fourth column 908 includes, for each processing task of the set of processing tasks, a preference for local processing vs. cloud processing of the processing task, based on the general estimates for bandwidth and computation for the processing task. Thus, using lookup table 900, a generally preferred location may be determined based on estimated bandwidth and computational demands. The generally preferred location may then be refined by a model such as model 800, by introducing different transmission latency conditions. In some examples, a user is able to edit the table according to their preferences.

Returning to FIG. 6, determining whether the data transmission time to candidate CCIs is within acceptable latency limits for transmitting the processing task to the candidate CCIs may first include retrieving from a memory of the resource allocation system a model (e.g., model 800) applicable to a type of scan being performed, and then applying the model to each CCI of the candidate CIs to determine whether the processing task should be preferentially performed locally or may be performed on the CCI. Each CCI may have a different data transmission time, whereby a same processing task may be advantageously performed on a first CCI, but may not be advantageously performed on a second CCI of the candidate CIs.

If at 608 it is determined that the data transmission times to one or more CCIs of the candidate CIs are not within the latency limits indicated by the model, method 600 proceeds to 610. At 610, method 600 includes eliminating the one or more CCIs from the candidate CIs for performing the processing task, thereby ensuring that the one or more CCIs are not selected for processing the processing task. Alternatively, if at 606 it is determined that the data transmission times to the CCIs of the candidate CIs are within the latency limits indicated by the model, method 600 proceeds to 612.

At 612, method 600 includes determining an availability of each candidate CI. The availability may be determined based on a demand for the candidate CI at a time that the processing task is to be executed (e.g., during an operational shift at the time that the processing task is to be executed). In various embodiments, an availability of a candidate CCI may be presumed. In some examples, the demand for a candidate LCI may be estimated from a historical use of the candidate LCI, for the day, time of day, and/or other factors. For example, the historical use of the candidate LCI may be tracked in a computer system of a healthcare system, and may indicate that the candidate LCI is typically in demand 90% of the time. Alternatively, the historical use may be estimated by averaging a percentage of time during which the candidate LCI was used on patient scans over a number of randomly selected past operational shifts of the candidate LCI.

In other examples, the demand may be calculated based on a schedule of use of the accessible LCI, meaning, a schedule of medical studies to be performed on patients using the accessible LCI during the operational shift. The schedule of use may be stored in a computer system of a healthcare system and/or retrieved via a network (e.g., network 250). The demand may be expressed as a percentage of time out of an operational shift during which the LCI is scheduled to be occupied. For example, a demand on an LCI may be 80%, where the LCI is scheduled to be used 80% of the time during a current operational shift for scanning patients. If the demand is above a threshold demand, the candidate CI may not be available. If the current demand is below the threshold demand, the candidate CI may be available for performing the processing task.

A weight value may then be assigned to each candidate CI, based on the calculated demand. The weight value may be a numerical value between 0 and 1, for example, where a lower weight value indicates a lower demand, and a higher weight value indicates a higher demand. For example, a first candidate CI may be in demand 80% of the time, and the candidate CI may be assigned a weight value of 0.8. A second candidate CI may be in demand 20% of the time, and the candidate CI may be assigned a weight value of 0.2.

At 614, method 600 includes calculating a suitability score of each candidate CI, where the suitability score is a sum of the data transmission time and the compute time for each candidate CI, multiplied by the weight value assigned to the candidate CI. The suitability score may indicate a relative suitability of each candidate CI for performing the processing task, taking into consideration the compute time of the CI, the time taken to transfer data to and from the CI to perform the processing task, and a relative demand on the CI.

At 616, method 600 includes selecting a CI of the candidate CIs having a lowest (e.g., minimum) suitability score for processing the task, and method 600 ends. By selecting the lowest suitability score (e.g., the minimum total weighted sum), the processing task may be assigned to the CI that is able to perform the processing task in the shortest amount of time.

For example, a first LCI may be used infrequently (e.g., in low demand), and the first LCI may be assigned a weight value of 0.2. A second LCI may be in higher demand, and the second LCI may be assigned a weight value of 0.6. The first LCI and the second LCI may have similar compute times and data transfer times. However, when the sum of the compute time and the data transfer time of the first LCI is multiplied by the weight value of 0.2, a resulting suitability score is less than a second suitability score of the second LCI based on the sum of the compute time and the data transfer time of the second LCI multiplied by the weight value of 0.6. As a result, the processing task may be assigned to the first, more available LCI. In this way, processing tasks may be assigned to both LCIs and CCIs in a manner that balances an overall processing load between all candidate CIs, and reduces an overall amount of time taken for performing all of the processing tasks.

Referring now to FIG. 7, an exemplary method 700 is shown for monitoring and dynamically reassigning one or more processing tasks between various candidate LCIs and CCIs of one or more imaging systems, based on data transmission times (e.g., latencies) between a primary LCI and the various accessible or available CIs. That is, one or more processing tasks may be assigned to a second CI other than the primary LCI at a first time, based on a data transmission time between a primary LCI and the second CI at the first time. However, at a second time during a processing of the task by the second CI, the data transmission time may have increased, for example, due to network traffic, bandwidth limitations, an amount of data generated during the processing, or a different factor. As a result of the increased data transmission time, the one or more processing tasks may be transferred to a different CI with a lower data transmission time, to reduce a total amount of time for processing the processing tasks. For example, a processing task may be assigned to a CCI, and then reassigned to an LCI as a result of a slow network connection to the CCI. Further, in some scenarios, the network connection may be lost, or the data transmission time may be so slow that a transfer of data may not be possible. In such cases, the processing task may be reassigned from the second CI to a different CI, such as the primary LCI. Method 700 may be performed as part of method 400 described above.

Method 700 begins at 702, where method 700 includes monitoring a first data transmission time for transmitting data associated with one or more processing tasks between the primary LCI associated with a scan performed using an imaging system of the one or more imaging systems, and the second CI on which the one or more processing tasks of the scan are being performed, in accordance with method 400. For example, the second CI may have a higher availability and/or greater processing power than the primary LCI, whereby the one or more processing tasks may be performed in a shorter amount of time on the second CI. Monitoring the first data transmission time may include periodically calculating the first data transmission time, based on an amount of the data associated with one or more processing tasks and a measured latency of network speeds.

At 704, method 700 includes determining whether the first transmission time is greater than a first threshold transmission time. The first threshold transmission time may be a predetermined maximum amount of time allowed or allocated for transferring the data associated with the one or more processing tasks back to the primary LCI. In some embodiments, the first threshold transmission time may be calculated based on the amount of the data of the one or more processing tasks and a predefined per-unit data transmission rate.

If at 704 it is determined that the first transmission time is not greater than the first threshold, method 700 proceeds to 709, to continue processing. Alternatively, if at 704 it is determined that the first transmission time is greater than the first threshold transmission time, it may be inferred that the one or more processing tasks are no longer most efficiently performed at the second CI, whereby method 700 proceeds to 706. At 706, method 700 includes recalculating suitability scores for the candidate CIs, and reassigning the one or more processing tasks from the second CI to the CI with the lowest suitability score (e.g., a CI with lower or negligible data transmission times), as described above in reference to FIG. 6.

In some scenarios, the recalculated suitability scores may indicate that no candidate CIs are suitable, except for the primary LCI. For example, a network connection may be lost, whereby the primary LCI may not be able to connect with any of the candidate CIs.

At 707, method 700 includes determining whether candidate CIs are available to perform the processing. If at 707 it is determined that candidate CIs are available to perform the processing, then method 700 proceeds to 709 to continue with processing. Alternatively, if at 707 it is determined that no candidate CIs are available to perform the processing, method 700 proceeds to 708.

At 708, method 700 includes reassigning and continuing processing of the one or more processing tasks at the primary/local LCI, using local, fallback versions of the models, parameters, and data. For example, an AI or ML model used in the execution of the one or more processing tasks at the second CI. In the event that a connection is lost between the primary LCI and the second CI, a local fallback version of the AI or ML model may be used to continue to process the one or more processing tasks at the primary LCI, as described above in reference to method 400, and method 700 ends.

At 709, method 700 includes monitoring a second data transmission time for transferring the model and parameter data used by the second CI for performing the one or more processing tasks to the primary LCI (or a different LCI within the same facility as the primary LCI). To ensure that the local versions of the AI or ML model are maintained up-to-date at the primary LCI, updated versions and/or parameters of the AI or ML model may be periodically transmitted back to the primary LCI or different LCI within the same facility as the primary LCI.

At 710, method 700 includes determining whether the second data transmission time is less than a second threshold. The second threshold may be a transmission time sufficiently low for opportunistically transferring the model and parameter data used by the second CI back to the primary LCI. In other words, the model and parameter data used by the second CI may be transferred to the primary LCI when the calculated data transmission time of the model and parameter data is estimated to be close to a minimum data transmission time.

If at 710 it is determined that the second data transmission time is less than the second threshold, method 700 proceeds to 712. At 712, method 700 includes updating the local fallback versions of models, parameters, and data of the second CI at the primary LCI or LCI within the same facility as the primary LCI. Alternatively, if at 712 it is determined that the second data transmission time is not less than the second threshold, method 700 proceeds to 714.

At 714, method 700 includes continuing to process the one or more processing tasks under the current resource allocation. That is, processing tasks assigned to the second CI may continue to be performed at the second CI, and processing tasks assigned to the primary LCI or an LCI within a same facility as the primary LCI may continue to be performed at those LCIs. Method 700 ends.

Thus, a framework is disclosed for increasing an efficiency of data processing tasks associated with generating a reconstructed image, including post-processing for diagnostic purposes, from scan data acquired via a medical imaging system, using a proposed resource allocation system. The resource allocation system identifies a series of individual processing tasks to perform, and then strategically allocates the individual processing tasks between a set of candidate CIs to balance computational loads, and increase scanner throughput by reducing an overall processing time for image reconstruction and automated diagnostic tasks. The individual processing tasks may be assigned priorities, and assigned to different candidate CIs based on the priorities. The priorities may be used in conjunction with, or may be generated based on calculated or estimated compute times for the different processing tasks and data transmission times between a scanner and each candidate CI. Additionally, the data transmission times may be monitored, and the processing tasks may be dynamically reassigned to maximize an overall efficiency of use of the set of candidate CIs. In this way, the functionality of the medical imaging system may be improved and increased, such that more patient scans may be performed, and a time spent on each patient exam may be reduced. The technical effect of allocating different compute instances to perform different processing tasks involved in performing a medical imaging exam in accordance with the resource allocation system described herein, is that by distributing the processing tasks based on resource availability and capability, the different processing and memory resources used may be maintained and managed more efficiently, leading to cost reductions, longer useful lives of computing components, and a reduction in overloaded machines.

The disclosure also provides support for a resource allocation system of a connected network of medical imaging systems, the resource allocation system comprising: a processor, and instructions stored in a memory of the resource allocation system that when executed, cause the processor to: receive a scan protocol, reconstruction instructions, and post-reconstruction image processing instructions for a scan to be performed on a first imaging system of the connected network of medical imaging systems, perform a first set of processing tasks of the scan at one or more local compute instances (LCIs) of the first imaging system, based on the scan protocol and the reconstruction instructions, perform a second processing task of the scan at a cloud compute instance (CCI) of the connected network of medical imaging systems, based on the post-reconstruction image processing instructions, the second processing task performed using an artificial intelligence (AI) model, and in response to a data transmission time between the first imaging system and the CCI being greater than a threshold data transmission time: sending parameter data of the AI model to a selected LCI of the one or more LCIs, terminating processing of the second processing task at the CCI, finishing the processing of the second processing task at the selected LCI, using a fallback version of the AI model installed at the selected LCI, and displaying the processed image on a display device. In a first example of the system, each processing task of the first set of processing tasks of the scan is assigned to an LCI of the one or more LCIs based on a sum of a calculated data transmission time of scan data used and/or generated during the processing task between the LCI and a scanner of the first imaging system, and a calculated compute time for performing the processing task on the LCI, weighted by a weight value based on an estimated demand for the LCI at a time that the processing task is to be executed. In a second example of the system, optionally including the first example, the estimated demand for the LCI at the time that the processing task is to be executed is a percentage of time out of an operational shift of the LCI during which the LCI is occupied, that is estimated based on at least one of: a historical use of the LCI, and a schedule of use of the LCI. In a third example of the system, optionally including one or both of the first and second examples, the schedule of use includes a schedule of medical studies to be performed on patients using the LCI during the operational shift. In a fourth example of the system, optionally including one or more or each of the first through third examples, the historical use of the LCI is estimated by: averaging a percentage of time during which the LCI was used on patient scans over a number of randomly selected past operational shifts of the LCI. In a fifth example of the system, optionally including one or more or each of the first through fourth examples, the one or more LCIs are selected by the resource allocation system without sending requests for service to the one or more LCIs.

The disclosure also provides support for a method for a resource allocation system of a connected network of medical imaging systems, the method comprising: receiving a scan protocol and reconstruction instructions for performing a scan on a first imaging system of the connected network of medical imaging systems, the first imaging system including a first local compute instance (LCI) for processing data acquired during the scan, determining a plurality of data processing tasks included in the protocol and reconstruction instructions, for each data processing task of the plurality of data processing tasks: determining a set of candidate compute instances (CIs) of the connected network of medical imaging systems that are accessible to the first LCI, for each candidate CI of the set of candidate CIs: calculating a data transmission time of scan data used and/or generated during the performance of the data processing task between the candidate CI and the first LCI, calculating a compute time for performing the data processing task on the candidate CI, calculating a sum of the data transmission time and the compute time for the candidate CI, calculating a weight value based on an estimated demand for the candidate CI at a time that the processing task is to be executed, multiplying the sum by the weight value, to generate a suitability score for the candidate CI, performing the data processing task on the CI with a lowest suitability score, and displaying an image reconstructed in accordance with the plurality of data processing tasks on a display device. In a first example of the method, one or more selected CIs include: a second LCI, either within a same health care facility as the first LCI, on a same internal healthcare network as the first LCI, or communicatively coupled to the first LCI via an external network, and a cloud compute instance (CCI) communicatively coupled to the first LCI via the Internet. In a second example of the method, optionally including the first example, the method further comprises: retrieving a priority of a data processing task from a reference table stored in a memory of the resource allocation system, determining that the priority is less than a threshold priority, and in response, selecting the first LCI. In a third example of the method, optionally including one or both of the first and second examples, the estimated demand for the candidate CI at the time that the processing task is to be executed is a percentage of time out of an operational shift of the CI during which the CI is occupied, that is estimated based on at least one of: a historical use of the candidate CI, and a schedule of medical studies to be performed on patients using the candidate CI during the operational shift. In a fourth example of the method, optionally including one or more or each of the first through third examples, the method further comprises: determining that the data transmission time of the CCI is greater than a data transmission time threshold using a decision tree model, and in response, removing the CCI from the set of candidate CIs, wherein the decision tree model includes a set of specific latency conditions that are applied to general, predefined estimations of available bandwidth and compute time stored in a lookup table. In a fifth example of the method, optionally including one or more or each of the first through fourth examples, the method further comprises: performing a data processing task of the plurality of data processing tasks on the CCI, using an artificial intelligence (AI) model, storing a fallback version of the AI model, including parameters and data of the AI model, at the first LCI, monitoring a first data transmission time of data associated with the data processing task, between the first LCI and the CCI, and in response to the first data transmission time being greater than a first threshold data transmission time, recalculating suitability scores for the candidate CIs and reassigning the processing task to a candidate CI with a lowest suitability score. In a sixth example of the method, optionally including one or more or each of the first through fifth examples, the method further comprises: in response to no candidate CIs being available to perform the data processing task, performing the data processing task on the first LCI using the fallback version of the AI model. In a seventh example of the method, optionally including one or more or each of the first through sixth examples, the method further comprises: calculating a second data transmission time of parameter data of the AI model used for processing the data processing task between the first LCI and the CCI, and in response to the second data transmission time being less than a second threshold data transmission time, transmitting the parameter data to the first LCI to update the fallback version of the AI model. In a eighth example of the method, optionally including one or more or each of the first through seventh examples, the method further comprises: for each candidate CI of the set of candidate CIs: estimating a financial cost of performing the data processing task on the candidate CI, and performing the data processing task on the CI with a combination of the lowest suitability score and a lowest financial cost. In a ninth example of the method, optionally including one or more or each of the first through eighth examples, a data processing task of the data processing tasks includes performing a post-reconstruction image processing task on an image reconstructed from projection data acquired using the first imaging system. In a tenth example of the method, optionally including one or more or each of the first through ninth examples, the post-reconstruction image processing task includes screening the image for health conditions using an AI detection model. In an eleventh example of the method, optionally including one or more or each of the first through tenth examples, the post-reconstruction image processing task includes processing the image as part of a multi-site federated learning scheme to train an AI model at the CCI. In a twelfth example of the method, optionally including one or more or each of the first through eleventh examples, performing the data processing task on the CI with the lowest suitability score further comprises assigning the data processing task to be performed on the CI without sending a prior request to the CI.

The disclosure also provides support for a resource allocation system of a connected network of medical imaging systems, the resource allocation system comprising: a processor, and instructions stored in a memory of the resource allocation system that when executed, cause the processor to: during a scan of a patient on an imaging system of the connected network of medical imaging systems, the imaging system including a first local compute instance (LCI) for processing data acquired during the scan, assign a data processing task included in a protocol of the scan to a different compute instance (CI) of a plurality of candidate CIs of the connected network of medical imaging systems, the CI selected based on a calculated data transmission time for sending data associated with the data processing task between the LCI and the CI, a calculated compute time for performing the data processing task on the CI, and an estimated availability of the CI, wherein the estimated availability of the CI is estimated based on a schedule of medical studies to be performed on patients using the CI during an operational shift at a time of performing the data processing task.

When introducing elements of various embodiments of the present disclosure, the articles "a," "an," and "the" are intended to mean that there are one or more of the elements. The terms "first," "second," and the like, do not denote any order, quantity, or importance, but rather are used to distinguish one element from another. The terms "comprising," "including," and "having" are intended to be inclusive and mean that there may be additional elements other than the listed elements. As the terms "connected to," "coupled to," etc. are used herein, one object (e.g., a material, element, structure, member, etc.) can be connected to or coupled to another object regardless of whether the one object is directly connected or coupled to the other object or whether there are one or more intervening objects between the one object and the other object. In addition, it should be understood that references to "one embodiment" or "an embodiment" of the present disclosure are not intended to be interpreted as excluding the existence of additional embodiments that also incorporate the recited features.

In addition to any previously indicated modification, numerous other variations and alternative arrangements may be devised by those skilled in the art without departing from the spirit and scope of this description, and appended claims are intended to cover such modifications and arrangements. Thus, while the information has been described above with particularity and detail in connection with what is presently deemed to be the most practical and preferred aspects, it will be apparent to those of ordinary skill in the art that numerous modifications, including, but not limited to, form, function, manner of operation and use may be made without departing from the principles and concepts set forth herein. Also, as used herein, the examples and embodiments, in all respects, are meant to be illustrative only and should not be construed to be limiting in any manner.

## Claims

1. A resource allocation system (105, 302) of a connected network (250) of medical imaging systems, the resource allocation system (105, 302) comprising:
a processor (304), and instructions stored in a memory of the resource allocation system (105, 302) that when executed, cause the processor (304) to:
receive a scan protocol, reconstruction instructions, and post-reconstruction image processing instructions for a scan to be performed on a first imaging system of the connected network (250) of medical imaging systems;
perform a first set of processing tasks of the scan at one or more local compute instances (LCIs (122, 112)) of the first imaging system, based on the scan protocol and the reconstruction instructions;
perform a second processing task of the scan at a second compute instance (CI) (108, 150, 162) of the connected network (250) of medical imaging systems, based on the post-reconstruction image processing instructions, the second processing task performed using an artificial intelligence (AI) model (800); and
in response to a data transmission time between the first imaging system and the second CI being greater than a threshold data transmission time:
sending parameter data of the AI model (800) to a selected LCI of the one or more LCIs (162, 152, 1004, 342, 132, 142);
reassigning the second processing task from the second CI to the selected LCI;
finishing the processing of the second processing task at the selected LCI, using a fallback version of the AI model (800) installed at the selected LCI; and
displaying the processed image on a display device (232, 334).

2. The resource allocation system (105, 302) of claim 1, wherein each processing task of the first set of processing tasks of the scan is assigned to an LCI (162, 152, 1004, 342, 122, 132, 142, 112, 1002) of the one or more LCIs (162, 152, 1004, 342, 122, 132, 142, 112, 1002) based on a sum of a calculated data transmission time of scan data used and/or generated during the processing task between the LCI (162, 152, 1004, 342, 122, 132, 142, 112, 1002) and a scanner (342, 344) of the first imaging system, and a calculated compute time for performing the processing task on the LCI (162, 152, 1004, 342, 122, 132, 142, 112, 1002), weighted by a weight value based on an estimated demand for the LCI (162, 152, 1004, 342, 122, 132, 142, 112, 1002) at a time that the processing task is to be executed.

3. The resource allocation system (105, 302) of claim 2, wherein the estimated demand for the LCI (162, 152, 1004, 342, 122, 132, 142, 112, 1002) at the time that the processing task is to be executed is a percentage of time out of an operational shift of the LCI (162, 152, 1004, 342, 122, 132, 142, 112, 1002) during which the LCI (162, 152, 1004, 342, 122, 132, 142, 112, 1002) is occupied, that is estimated based on at least one of:
a historical use of the LCI (162, 152, 1004, 342, 122, 132, 142, 112, 1002); and
a schedule of use of the LCI (162, 152, 1004, 342, 122, 132, 142, 112, 1002).

4. The resource allocation system (105, 302) of claim 3, wherein the schedule of use includes a schedule of medical studies to be performed on patients using the LCI (162, 152, 1004, 342, 122, 132, 142, 112, 1002) during the operational shift.

5. The resource allocation system (105, 302) of claim 3, wherein the historical use of the LCI (162, 152, 1004, 342, 122, 132, 142, 112, 1002) is estimated by:
averaging a percentage of time during which the LCI (162, 152, 1004, 342, 122, 132, 142, 112, 1002) was used on patient scans over a number of randomly selected past operational shifts of the LCI (162, 152, 1004, 342, 122, 132, 142, 112, 1002).

6. The resource allocation system (105, 302) of claim 2, wherein the one or more LCIs (162, 152, 1004, 342, 122, 132, 142, 112, 1002) are selected by the resource allocation system (105, 302) without sending requests for service to the one or more LCIs (162, 152, 1004, 342, 122, 132, 142, 112, 1002).

7. A method (400, 500, 600, 700) for a resource allocation system of a connected network of medical imaging systems, the method (400, 500, 600, 700) comprising:
receiving a scan protocol and reconstruction instructions for performing a scan on a first imaging system of the connected network of medical imaging systems (402), the first imaging system including a first local compute instance for processing data acquired during the scan;
determining a plurality of data processing tasks included in the protocol and reconstruction instructions (404);
for each data processing task of the plurality of data processing tasks:
determining a set of candidate compute instances (CIs) of the connected network of medical imaging systems that are accessible to the first LCI (510);
for each candidate CI of the set of candidate CIs:
calculating a data transmission time of scan data used and/or generated during the performance of the data processing task between the candidate CI and the first LCI (604);
calculating a compute time for performing the data processing task on the candidate CI (606);
calculating a sum of the data transmission time and the compute time for the candidate CI (614);
calculating a weight value based on an estimated demand for the candidate CI at a time that the processing task is to be executed;
multiplying the sum by the weight value, to generate a suitability score for the candidate CI (614);
selecting a candidate CI with a lowest suitability score (616);
performing the data processing task on the selected CI; and
displaying an image reconstructed in accordance with the plurality of data processing tasks on a display device (412).

8. The method (400, 500, 600, 700) of claim 7, wherein the selected CI is one of:
a second LCI, either within a same health care facility as the first LCI, on a same internal healthcare network as the first LCI; or communicatively coupled to the first LCI via an external network; and
a cloud compute instance (CCI) communicatively coupled to the first LCI via the Internet.

9. The method (400, 500, 600, 700) of claim 8, further comprising:
retrieving a priority of a data processing task from a reference table stored in a memory of the resource allocation system (504);
determining that the priority is less than a threshold priority (506), and in response, selecting the first LCI (508).

10. The method (400, 500, 600, 700) of claim 8, wherein the estimated demand for the candidate CI at the time that the processing task is to be executed is a percentage of time out of an operational shift of the candidate CI during which the candidate CI is occupied, that is estimated based on at least one of:
a historical use of the candidate CI; and
a schedule of medical studies to be performed on patients using the candidate CI during the operational shift.

11. The method (400, 500, 600, 700) of claim 8, further comprising:
determining that the data transmission time of the CCI is greater than a data transmission time threshold (608) using a decision tree model, and in response, removing the CCI from the set of candidate CIs (610);
wherein the decision tree model includes a set of specific latency conditions that are applied to general, predefined estimations of available bandwidth and compute time stored in a lookup table.

12. The method (400, 500, 600, 700) of claim 8, further comprising:
performing a data processing task of the plurality of data processing tasks on the CCI, using an artificial intelligence (AI) model;
storing a fallback version of the AI model, including parameters and data of the AI model, at the first LCI;
monitoring a first data transmission time of data associated with the data processing task, between the first LCI and the CCI (702); and
in response to the first data transmission time being greater than a first threshold data transmission time (704), recalculating suitability scores for the candidate CIs (706) and reassigning the processing task to a candidate CI with a lowest suitability score (708).

13. The method (400, 500, 600, 700) of claim 12, further comprising:
in response to no candidate CIs being available to perform the data processing task, performing the data processing task on the first LCI using the fallback version of the AI model.

14. The method (400, 500, 600, 700) of claim 12, further comprising:
calculating a second data transmission time of parameter data of the AI model used for processing the data processing task between the first LCI and the CCI; and
in response to the second data transmission time being less than a second threshold data transmission time (710), transmitting the parameter data to the first LCI to update the fallback version of the AI model (712).

15. The method (400, 500, 600, 700) of claim 7, further comprising:
for each candidate CI of the set of candidate CIs:
estimating a financial cost of performing the data processing task on the candidate CI; and
performing the data processing task on the CI with a combination of the lowest suitability score and a lowest financial cost.
